# EUROPEAN PATENT SPECIFICATION

(11) **EP 4 636 441 B1**
(45) Date of publication and mention of the grant of the patent: **23.09.2026**
(21) Application number: 25168369.4
(22) Date of filing: 03.04.2025
(51) Int. Cl.: G01T 1/20, G01T 1/24, A61B 6/00, A61B 6/03, A61B 6/42

(54) **BIASED DETECTOR SUB-MODULE, DETECTOR MODULE, DETECTOR, AND MEDICAL IMAGING DEVICE**
UNTERMODUL MIT VORGESPANNTEM DETEKTOR, DETEKTORMODUL, DETEKTOR UND MEDIZINISCHE BILDGEBUNGSVORRICHTUNG
SOUS-MODULE DE DÉTECTEUR POLARISÉ, MODULE DE DÉTECTEUR, DÉTECTEUR ET DISPOSITIF D'IMAGERIE MÉDICALE

(30) Priority: 16.04.2024 CN 202410459917
(43) Date of publication of application: 22.10.2025
(73) Proprietor: Neusoft Medical Systems Co., Ltd., Hunnan District Shenyang Liaoning 110167 (CN)
(72) Inventor: YU, Jun, Shenyang, 110167 (CN); CUI, Shifeng, Shenyang, 110167 (CN)
(74) Representative: Lewis Silkin LLP

(56) References cited:
- EP-A2- 3 789 798
- US-A1- 2002 130 266
- US-A1- 2012 193 545
- US-A1- 2015 247 936
- US-A1- 2020 121 270

## Description

### FIELD

The present disclosure relates to the field of medical equipment technologies, and more particularly, to a biased detector sub-module, a detector module, a detector, and a medical imaging device.

### BACKGROUND

In CT technologies, a plurality of biased detector sub-modules are arranged to form a detector module, where the direction in which the plurality of biased detector are arranged is referred to as the Z direction. In this direction, existing biased detector sub-modules cause a problem of a large stacking height difference ΔH when stacked. Due to thickness accumulation of components such as the substrate, the biased analog-to-digital converter, the protective tungsten plate, and the thermal conductive adhesive, distances from the biased detector sub-modules of different layers after stacking to a focal spot of an X-ray tube are different, causing the same pixel size to present different sizes at a scanning center, which not only increases image processing complexity, but also introduces errors, affecting the accuracy and reliability of CT images. Therefore, how to effectively reduce the stacking height difference ΔH and ensure that the biased detector sub-modules of different layers has a consistent pixel size at the scanning center has become an important technical problem faced by current CT detector module design.
EP3789798A2 discloses a detector module. The detector module includes: a support and a plurality of detector sub-modules arranged on the support along an extension direction in which the support extends. Each of the detector sub-modules has a first area and a second area in the extension direction. A detecting device is disposed in the first area, and a functional module is disposed in the second area. The functional module is electrically connected to the detecting device for receiving an electrical signal from the detecting device. The plurality of detector sub-modules includes a first detector sub-module and a second detector sub-module that are arranged adjacent to each other in the extension direction, and the first area of the first detector sub-module at least partially overlaps with the second area of the second detector sub-module.
US2012/193545A1 discloses a detector module for an imaging system. The detector module includes an array of direct conversion sensors, the direct conversion sensors having a first side and a second side. The first side of the direct conversion sensors includes a segmented electrode side forming an array of pixels that receive radiation and convert the received radiation into corresponding charge signals, whereas the second side includes a common electrode side. The detector module also includes a readout electronic circuitry coupled to one or more of the direct conversion sensors where the readout electronic circuitry is configured to be shielded from the radiation. In addition, the detector module includes a bias voltage circuitry coupled to the one or more direct conversion sensors on the second side.

### SUMMARY

The present disclosure aims to solve at least one of the technical problems in the related art. To this end, an objective in a first aspect of the present disclosure is to provide a biased detector sub-module. The biased detector sub-module has a smaller height difference when stacked, which reduces difficulty of image data error processing when splicing biased detector sub-modules in the Z direction to form a detector module with a large coverage area, improving image accuracy and reliability.

An objective in a second aspect of the present disclosure is to provide a detector module.

An objective in a third aspect of the present disclosure is to provide a detector.

An objective in a fourth aspect of the present disclosure is to provide a medical imaging device.

In a first aspect, embodiments of the present disclosure provide a biased detector sub-module including a photoelectric conversion array, a biased analog-to-digital converter, and a substrate. The photoelectric conversion array is arranged in an X direction and a Z direction. A plurality of biased detector sub-modules are stacked in the Z direction to form a detector module. The biased analog-to-digital converter is electrically connected to the photoelectric conversion array. The substrate includes a mounting substrate and a circuit connection substrate stacked in a Y direction. The mounting substrate has a first substrate end and a second substrate end opposite to each other in the Z direction, and the circuit connection substrate has a first substrate end and a second substrate end opposite to each other in the Z direction, where the first substrate end of the circuit connection substrate is adjacent to the first substrate end of the mounting substrate, and extends beyond the first substrate end of the mounting substrate, the second substrate end of the mounting substrate is adjacent to the second substrate end of the circuit connection substrate, and extends beyond the second substrate end of the circuit connection substrate. The photoelectric conversion array and the biased analog-to-digital converter are sequentially disposed in the Z direction at a side of the mounting substrate facing away from the circuit connection substrate. The biased analog-to-digital converter is adjacent to the first substrate end of the mounting substrate. A part of the mounting substrate that is adjacent to the second substrate end of the mounting substrate and that is not overlapped with the circuit connection substrate is configured to be stacked on an adjacent biased detector sub-module. The biased analog-to-digital converter is provided with a power connection portion at an end of the biased analog-to-digital converter away from the photoelectric conversion array, where the power connection portion is electrically connected to a part of the circuit connection substrate that is adjacent to the first substrate end of circuit connection substrate and that is not overlapped with the mounting substrate.

With the biased detector sub-module according to the embodiments of the present disclosure, the biased analog-to-digital converter and the photoelectric conversion array are arranged in the Z direction and connected to an end of the photoelectric conversion array. In this way, the biased analog-to-digital converter can be closer to the photoelectric conversion array, which not only reduces a distance between the biased analog-to-digital converter and the photoelectric conversion array and shortens a wire length, but also helps to reduce an overall height. The mounting substrate is partially overlapped with the circuit connection substrate, in such a manner that the part of the mounting substrate that is not overlapped with the circuit connection substrate forms an avoidance space for accommodating an adjacent biased detector sub-module, so as to shorten a height difference between two biased detector sub-modules after stacked in the Z direction. Therefore, the different in radii from the detector sub-modules of different layers to a focal spot of an X-ray tube is reduced, reducing an influence of a pixel size difference on imaging.

With the biased detector sub-module according to some embodiments of the present disclosure, the photoelectric conversion array has a first tube end and a second tube end in the Z direction, and the biased analog-to-digital converter is located adjacent to the first tube end. At least part of the biased analog-to-digital converter is located between the first substrate end and the second substrate end of the circuit connection substrate. A distance L1 between the first substrate end of the circuit connection substrate and the first tube end in the Z direction and a distance L2 between the second substrate end of the circuit connection substrate and the second tube end in the Z direction satisfies L1<L2.

With the biased detector sub-module according to some embodiments of the present disclosure, the mounting substrate is made of a high-rigidity material.

In some embodiments, the mounting substrate is a ceramic substrate or a stainless steel substrate. A dimension W1 of the mounting substrate in the Y direction satisfies 0.2 mm ≤ W1 ≤ 2.5 mm.

The biased detector sub-module according to some embodiments of the present disclosure further includes a protective plate disposed at a side of the biased analog-to-digital converter facing away from the circuit connection substrate and configured to shield radiation.

In some embodiments, the protective plate has an avoidance groove configured to avoid the power connection portion.

In some alternative embodiments, a dimension W2 of the avoidance groove in the Y direction satisfies 0.3 mm ≤ W2 ≤ 1 mm. A dimension W3 of the protective plate in the Y direction satisfies 0.8 mm ≤ W3 ≤ 2 mm.

The biased detector sub-module according to some embodiments of the present disclosure further includes a connector electrically connected to the circuit connection substrate. The connector is a rigid member or a flexible member. The connector and the circuit connection substrate form a rigid-flex board.

With the biased detector sub-module according to some embodiments of the present disclosure, the biased analog-to-digital converter and the photoelectric conversion array are disposed at a same chip.

In a second aspect, a detector module according to embodiments of the present disclosure includes a module support and a plurality of biased detector sub-modules. The plurality of biased detector sub-modules are the biased detector sub-module according to the embodiments in the first aspect of the present disclosure. The plurality of biased detector sub-modules are mounted at the module support and sequentially arranged in the Z direction. The plurality of biased detector sub-modules include at least two biased detector sub-modules sequentially stacked in the Z direction. For two adjacent biased detector sub-modules that are stacked, a part of the mounting substrate of one of the two adjacent biased detector sub-modules that is adjacent to the second substrate end of the mounting substrate and that is not overlapped with the circuit connection substrate is stacked on the circuit connection substrate of the other of the two adjacent biased detector sub-modules.

In a third aspect, a detector according to embodiments of the present disclosure includes a housing and a plurality of detector modules according to embodiments in the second aspect of the present disclosure. The plurality of the detector modules are arranged in parallel at the housing in the X direction.

In a fourth aspect, a medical imaging device according to the embodiments of the present disclosure includes a scanning frame, a radiation source, and the detector according to embodiments in the third aspect of the present disclosure. The scanning frame is configured to accommodate a scanning object. The radiation source and the detector each are disposed at the scanning frame. The radiation source is configured to emit rays to the scanning object. The detector is configured to receive rays attenuated by the scanning object.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and more understandable from the following description of embodiments taken in conjunction with the accompanying drawings.
FIG. 1 is a schematic diagram showing positions of a detector and a radiation source in the related art.
FIG. 2 is a schematic structural diagram of a biased detector sub-module according to the wording of the claims.
FIG. 3 is a schematic diagram showing an arrangement principle of sub-modules in a detector module according to some embodiments of the present disclosure.
FIG. 4 is a schematic diagram showing an arrangement of a photoelectric conversion array and a biased analog-to-digital converter according to some embodiments of the present disclosure.
FIG. 5 is a schematic structural diagram of a substrate according to the wording of the claims.
FIG. 6 is a schematic structural diagram of a protective plate according to some embodiments of the present disclosure.
FIG. 7 is a schematic diagram showing an arrangement of sub-modules in a detector module according to some embodiments of the present disclosure.
FIG. 8 is a schematic diagram showing an arrangement of a first biased detector sub-module and a second biased detector sub-module according to some embodiments of the present disclosure.
FIG. 9 is a schematic diagram showing an arrangement of a first biased detector sub-module and a second biased detector sub-module according to some embodiments of the present disclosure.
FIG. 10 is a schematic structural diagram of a medical imaging device according to some embodiments of the present disclosure.

Reference numerals of the accompanying drawings:
detector 10' in the related art, focal spot of X-ray tube Q0', radiation source 30';
medical imaging device 100 of the present disclosure;
detector 10, scanning frame 20, scanning cavity 201, radiation source 30, focus of the radiation source Q0, scanning object 40, scanning bed 50;
detector module 101, module support 102;
biased detector sub-module 103, first biased detector sub-module 1031, second biased detector sub-module 1032;
photoelectric conversion array 1, first tube end 11, second tube end 12, biased analog-to-digital converter 2, power connection portion 22, substrate 3, mounting substrate 31, circuit connection substrate 32, first substrate end 321, second substrate end 322, connector 4, protective plate 5, avoidance groove 51, avoidance position 7.

### DETAILED DESCRIPTION

Embodiments of the present disclosure will be described in detail below with reference to examples thereof as illustrated in the accompanying drawings, throughout which same or similar elements, or elements having same or similar functions, are denoted by same or similar reference numerals. The embodiments described below with reference to the drawings are illustrative only, and are intended to explain, rather than limit, the present disclosure.

In the description of the present disclosure, it should be understood that the orientation or the positional relationship indicated by terms such as "length", "width", "thickness", "over", "below", "front", "rear", "top", "bottom", "inner", "outer", "axial" and "radial" should be construed to refer to the orientation or the positional relationship as shown in the drawings, and is only for the convenience of describing the present disclosure and simplifying the description, rather than indicating or implying that the involved device or element must have a specific orientation, or be constructed and operated in a specific orientation, and therefore cannot be understood as a limitation of the present disclosure. In addition, the features associated with "first" and "second" may explicitly or implicitly include at least one of the features. In the description of the present disclosure, unless otherwise specified, "a plurality" means two or more.

In the description of the present disclosure, it should be noted that, unless otherwise clearly specified and limited, terms such as "install", "couple", "connect", and the like should be understood in a broad sense. For example, a connection may be a fixed connection or a detachable connection or connection as one piece, mechanical connection or electrical connection, direct connection or indirect connection through an intermediate, or internal communication of two components. For those of ordinary skill in the art, the specific meaning of the above-mentioned terms in the present disclosure can be understood according to specific circumstances.

A detector is formed by detector modules 101. The detector 10 is configured to detect rays emitted by a radiation source and attenuated by a scanning object 40. Therefore, each detector module 101 is also configured to detect the rays emitted by the radiation source and attenuated by the scanning object 40. The detector 10 is applicable to any device, and may be applied to a medical imaging device 100 or other devices that require scanning imaging.

Taking the medical imaging device 100 being a Computed Tomography (CT) scanner as an example, with development of the CT scanner, the number of detector layers in the CT scanner is increasing. To facilitate manufacturing and improve a yield rate, the detector is usually divided into dozens of detector modules in an X direction, that is, a channel direction, and these detector modules are disposed at an arc centered at a focal spot. Each detector module is divided into one to dozens of detector sub-modules in a Z direction, that is, a layer arrangement direction, or along a rotation axis of the CT scanner according to the number of layers required. Usually, each detector sub-module has matrix of 32 × 6 detector units, or 32 × 32 detector modules, etc.

An existing detector sub-module usually includes a scintillator array, a photodiode array, a substrate, a connection line, a circuit board, an analog-to-digital converter, etc. The detector sub-module completes conversion of rays, such as X-rays, into electrical signals. The scintillator array usually has a matrix structure of 32 × 16, or 16 × 16, etc. The detector sub-module may have a simplified structure consisting only of the scintillator array, the photodiode array, and the substrate, and additionally connected to the analog-to-digital converter through a connector.

However, such detector sub-modules still has a large height difference ΔH after being stacked in the Z direction, where ΔH = a thickness of the substrate + a height of the analog-to-digital converter + a height of a protective tungsten plate + a thickness of a thermal conductive adhesive. The height difference ΔH causes different radii from the detector sub-modules of two layers to a focal spot of an X-ray tube Q0', further causing the same detector sub-module pixel size to correspond to different sizes at a scanning center. This difference not only increases complexity of image processing, but also affects accuracy and consistency of imaging.

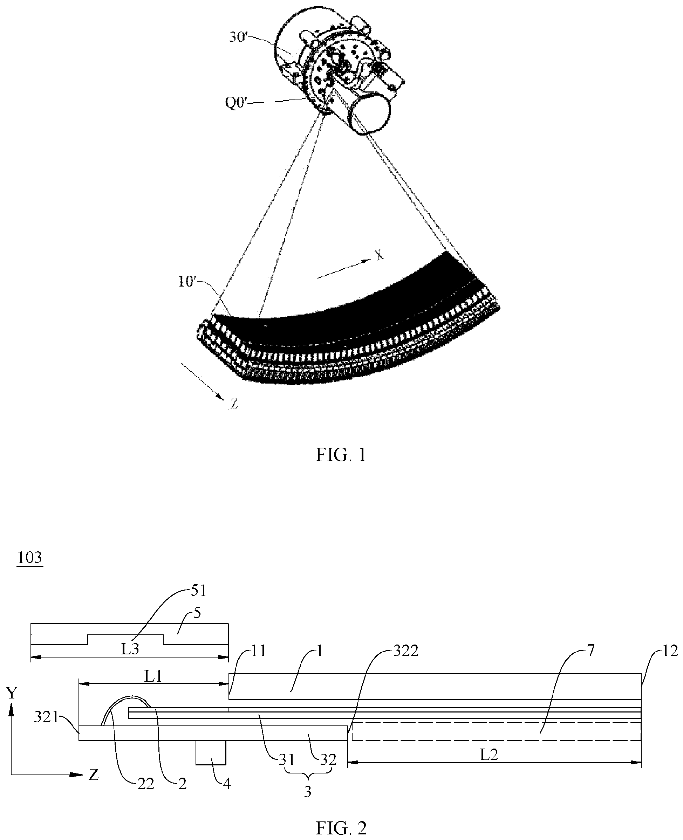

To solve the above problems, a biased detector sub-module 103 is provided according to embodiments in a first aspect of the present disclosure, which is described below with reference to FIG. 2 to FIG. 10.

As illustrated in FIG. 2, the biased detector sub-module 103 according to the embodiments of the present disclosure includes a photoelectric conversion array 1, a biased analog-to-digital converter 2, and a substrate 3.

The photoelectric conversion array 1 is arranged in an X direction and a Z direction.

It is to be noted that, the photoelectric conversion array 1 includes a scintillator array and a photodiode array stacked in a Y direction.

The scintillator array is disposed at a side of the photoelectric conversion array 1 facing away from the substrate 3 and arranged in the X direction and the Z direction.

The scintillator array functions to absorb rays and convert the rays into visible light or ultraviolet light. When the rays pass through a scanning object 40 and enter the scintillator array, scintillators absorb energy in the rays and emits photons. The photons propagate in the Y direction and enter the photodiode array closely adjacent to the scintillator array. Photodiodes in the photodiode array can receive these photons and convert the photons into electrical signals, realizing detection and imaging of the rays.

In addition, the scintillator array is arranged in the X direction and the Z direction to cover a detection region of the photodiode, such that the photons are not required to change directions or pass through additional media. The direct photoelectric conversion path reduces loss and scattering of the photons, improving resolution and clarity of imaging.

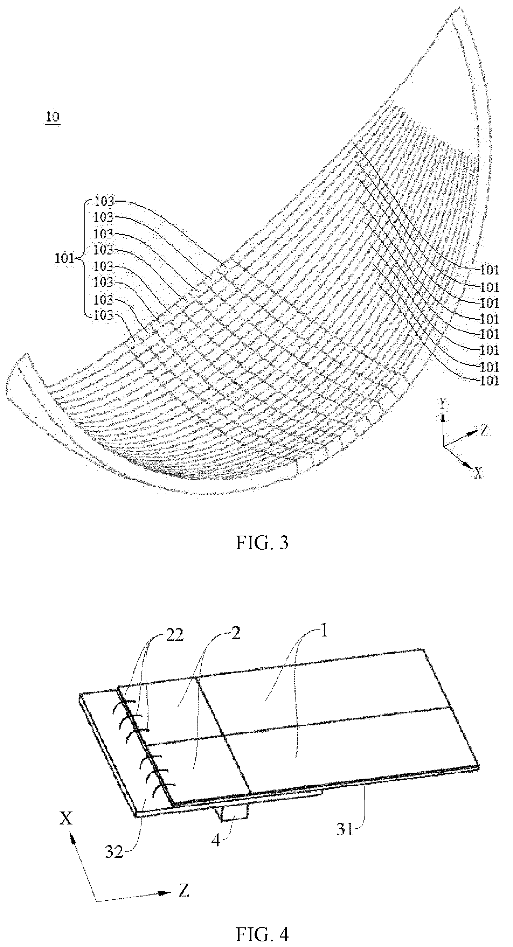

As illustrated in FIG. 3, a plurality of biased detector sub-modules 103 are arranged in the Z direction to form the detector module 101. In this way, arrangement of the photoelectric conversion array 1 can ensure coverage of the detector module 101 in the Z direction. The X direction forms an angle with the Z direction. The photoelectric conversion array 1 is extended in the X direction.

As illustrated in FIG. 4, the biased analog-to-digital converter 2 is electrically connected to the photoelectric conversion array 1. The biased analog-to-digital converter 2 functions to convert an analog signal generated by the photoelectric conversion array 1 into a digital signal for subsequent data processing and analysis.

The biased analog-to-digital converter 2 and the photoelectric conversion array 1 are arranged in the Z direction. The biased analog-to-digital converter 2 is arranged adjacent to the photoelectric conversion array 1, to ensure signal transmission efficiency and stability. By arranging in the Z direction, the biased analog-to-digital converter 2 can closely follow the photoelectric conversion array 1, to shorten a wire length for the analog signal to the biased analog-to-digital converter 2, and further reduce background noise, which realizes reduction of loss and interference in a signal transmission process, improving signal quality.

In the present disclosure, the biased analog-to-digital converter 2 refers to an analog-to-digital converter located at a side of the photoelectric conversion array 1 in the Z direction. Of course, the present disclosure does not exclude that the analog-to-digital converter further includes a stacked analog-to-digital converter. When the stacked analog-to-digital converter is provided, the stacked analog-to-digital converter is disposed between the photoelectric conversion array 1 and the mounting substrate 31.

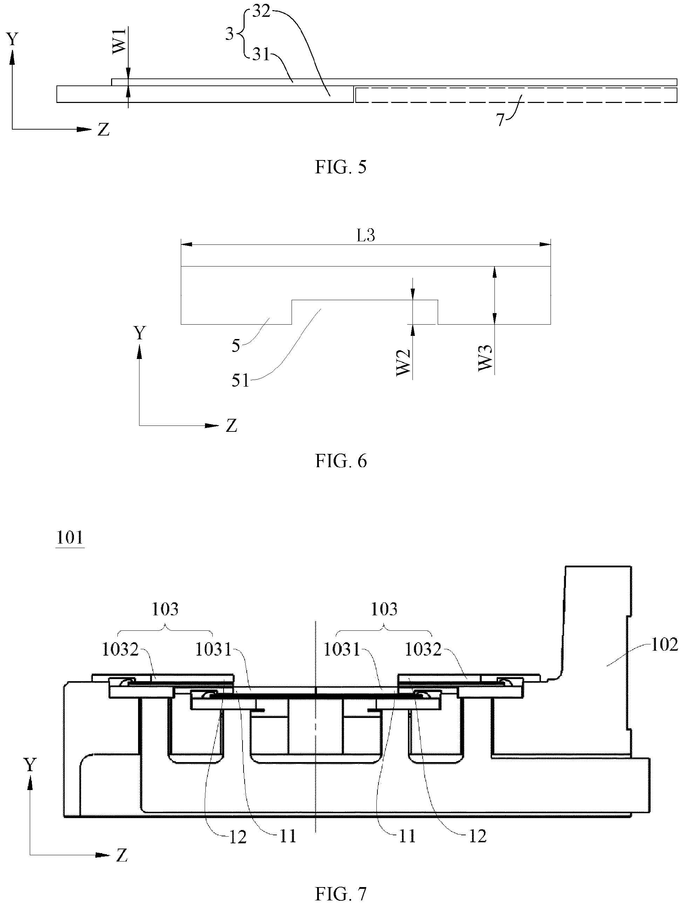

As illustrated in FIG. 2 and FIG. 5, the substrate 3 includes a mounting substrate 31 and a circuit connection substrate 32 stacked in the Y direction. The circuit connection substrate 32 is electrically connected to the biased analog-to-digital converter 2. The substrate 3 not only serves as a supporting structure for the entire biased detector sub-module 103, but also transmits the electrical signal generated by the photoelectric conversion array 1 to the biased analog-to-digital converter 2 by adopting the CMOS integrated circuit technology instead of traditional connection lines and circuit boards. Therefore, the entire substrate 3 has a more compact structure, and the signal transmission distance is shortened, which correspondingly reduces interference factors in the signal transmission process, facilitating improvement of image quality.

As illustrated in FIG. 4, the mounting substrate 31 is configured to support the photoelectric conversion array 1 to ensure stability and reliability of the photoelectric conversion array 1.

As illustrated in FIG. 4, the circuit connection substrate 32 functions to carry an electrical structure including an electrical connection part electrically connected to the biased analog-to-digital converter 2, in such a manner that signals can be smoothly transmitted to an external device.

In an embodiment, a conventional printed circuit board (PCB), such as an FR4 (epoxy glass fiber) board or a ceramic PCB, is used as the circuit connection substrate 32. The FR4 board has good electrical properties, mechanical properties, and processing properties. The FR4 board can meet basic requirements of the detector 10 in terms of circuit connection, and simultaneously has relatively low costs, which is suitable for mass production and application. The ceramic PCB is a high-performance circuit connection substrate 32 with excellent high temperature resistance, corrosion resistance, and electrical properties, enabling the biased detector sub-module 103 to have good performance.

In an embodiment, a part of the mounting substrate 31 that is overlapped with the circuit connection substrate 32 is connected to the circuit connection substrate 32 by an adhesive layer.

As illustrated in FIG. 2, the photoelectric conversion array 1 and the biased analog-to-digital converter 2 are sequentially disposed in the Z direction at a side of the mounting substrate 31 facing away from the circuit connection substrate 32, and the biased analog-to-digital converter 2 is adjacent to an end portion of the mounting substrate 31 overlapping with the circuit connection substrate 32. A part of the mounting substrate 31 that is not overlapped with the circuit connection substrate 32 is configured to be stacked on an adjacent biased detector sub-module 103.

As illustrated in FIG. 2, the biased analog-to-digital converter 2 is adjacent to the end portion of the mounting substrate 31 overlapping with the circuit connection substrate 32. In this way, the biased analog-to-digital converter 2 is mounted more closely to the substrate 3, reducing space waste.

As illustrated in FIG. 5, the mounting substrate 31 is partially overlapped with the circuit connection substrate 32, in such a manner that a part of the mounting substrate 31 that is not overlapped with the circuit connection substrate 32 and an end of the circuit connection substrate 32 form an avoidance position 7. The avoidance position 7 is located below the mounting substrate 31, which provides accommodation space for part of the adjacent biased detector sub-module 103. This arrangement facilitates stacking and connection of the biased detector sub-modules 103.

As illustrated in FIG. 2, in the biased detector sub-module 103 according to some embodiments of the present disclosure, the photoelectric conversion array 1 has a first tube end 11 and a second tube end 12 in the Z direction. The biased analog-to-digital converter 2 is located adjacent to the first tube end 11. The circuit connection substrate 32 has a first substrate end 321 and a second substrate end 322 in the Z direction. At least part of the biased analog-to-digital converter 2 is located between the first substrate end 321 and the second substrate end 322. A distance L1 between the first substrate end 321 and the first tube end 11 in the Z direction and a distance L2 between the second substrate end 322 and the second tube end 12 in the Z direction satisfies L1<L2.

In an embodiment, in the biased detector sub-module 103, a length of the avoidance position 7 is L2. At another end of the biased detector sub-module 103, the photoelectric conversion array 1 is staggered from another end of the circuit connection substrate 32 in the Z direction by a distance of L1. Through this biased structural arrangement, a part of the biased detector sub-module 103 is contracted in the Y direction to provide space for stacked installation with an adjacent sub-module.

During installation of two adjacent biased detector sub-modules 103 in the Y direction, the L1 length part of one of the two adjacent biased detector sub-modules 103 can exactly extend to the avoidance position 7 with the L2 length of the other biased detector sub-module 103. Therefore, the two biased detector sub-modules 103 can be tightly stacked together without generating an excessive height difference in the Y direction.

Through this structure of the biased detector sub-module 103, connection between the biased detector sub-modules 103 is made tighter. Also, the height difference between the biased detector sub-modules 103 after splicing is relatively small, which can ensure that the difference in the radii from the biased detector sub-modules 103 of different layers to a focal spot of an X-ray tube is reduced. In this way, an influence of a pixel size difference on imaging is reduced, reducing difficulty of image processing, and improving accuracy and consistency of CT images.

With the biased detector sub-module 103 according to some embodiments of the present disclosure, the mounting substrate 31 is made of a high-rigidity material.

Using the substrate made of the high-rigidity material as the mounting substrate 31 of the biased detector sub-module 103 can effectively resist external stress and deformation, which ensures that the biased detector sub-module 103 has good flatness, improving stability of the detector module 101.

In some embodiments as illustrated in FIG. 5, the mounting substrate 31 is a ceramic substrate or a stainless steel substrate. A dimension W1 of the mounting substrate 31 in the Y direction satisfies 0.2 mm ≤ W1 ≤ 2.5 mm. For example, W1 is 0.2 mm, 0.3 mm, 0.5 mm, 1.5 mm, 2 mm, 2.5 mm, etc.

In an embodiment, the mounting substrate 31 is a ceramic substrate, a stainless steel substrate, or the like with a thickness of 0.3 mm to 1 mm.

In the biased detector sub-module 103 according to some embodiments of the present disclosure, as illustrated in FIG. 2 and FIG. 6, the biased detector sub-module 103 further includes a protective plate 5. The protective plate 5 is disposed at a side of the biased analog-to-digital converter 2 facing away from the circuit connection substrate 32 and configured to shield radiation.

As illustrated in FIG. 2, the protective plate 5 is staggered from the photoelectric conversion array 1 in the Z direction.

The protective plate 5 is disposed at the side of the biased analog-to-digital converter 2 facing away from the circuit connection substrate 32, in such a manner that the protective plate 5 can protect the biased analog-to-digital converter 2 and reduce exposure of the biased analog-to-digital converter 2 to interference and damage from the external environment. In addition, the protective plate 5 is staggered from the photoelectric conversion array 1 in the Z direction, which further makes the structure of the entire biased detector sub-module 103 more compact and reasonable.

It is to be noted that the protective plate 5 plays a dual role. On the one hand, the protective plate 5 has radiation shielding performance, which can effectively prevent radiation from damaging the biased analog-to-digital converter 2, ensuring normal operation of the biased analog-to-digital converter 2. On the other hand, the protective plate 5 can also effectively conduct heat generated by the biased analog-to-digital converter 2. The protective plate 5 is in contact with the module support 102 to realize heat dissipation, improving operation stability of the biased detector sub-module 103.

In some embodiments not shown in the figure, a thermal conductive adhesive is disposed between the biased analog-to-digital converter 2 and the protective plate 5. The thermal conductive adhesive facilitates timely transfer of the heat of the biased analog-to-digital converter 2 with large heat generation. At the same time, the thermal conductive adhesive also has moisture-proof and shock-proof functions.

In some embodiments as illustrated in FIG. 2, the biased analog-to-digital converter 2 is provided with a power connection portion 22 electrically connected to the circuit connection substrate 32 at an end of the biased analog-to-digital converter 2 away from the photoelectric conversion array 1. The biased analog-to-digital converter 2 is connected to the circuit connection substrate 32 through the power connection portion 22 to realize electrical signal transmission.

In an embodiment, the power connection portion 22 may be reliably connected to the circuit connection substrate 32 in a form of a binding wire connection, welding, or the like.

As illustrated in FIG. 2 and FIG. 6, the protective plate 5 has an avoidance groove 51 configured to avoid the power connection portion 22. Therefore, the protective plate 5 can protect the biased analog-to-digital converter 2 from radiation damage while avoiding interference to the power connection portion 22. In this way, the power connection portion 22 can be electrically connected to the circuit connection substrate 32 stably, ensuring transmission quality of the electrical signals.

In some embodiments, as illustrated in FIG. 2 and FIG. 6, a length L3 of the protective plate 5 in the Z direction is smaller than L2, which can ensure the compactness of the biased detector sub-modules 103 when spliced. When the biased detector sub-module 103 is mounted with the protective plate 5, since L3 is smaller than L2, the protective plate 5 can smoothly enter the space defined by L2, ensuring tight contact between the plurality of biased detector sub-modules 103 when spliced and avoiding the problem of loose splicing or excessive gaps due to a large protective plate 5.

In an embodiment, a dimension W2 of the avoidance groove 51 in the Y direction satisfies 0.3 mm ≤ W2 ≤ 1 mm. In this way, the avoidance groove 51 has a predetermined space for accommodating the power connection portion 22, such as a binding wire, to ensure that the binding wire can be electrically connected smoothly without being hindered by the protective plate 5. At the same time, the avoidance groove 51 is provided without weakening an overall structural strength of the protective plate 5 too much. W2 may be 0.3 mm, 0.5 mm, 0.8 mm, 1 mm, etc.

In some alternative embodiments, the protective plate 5 is a tungsten plate, and a dimension W3 of the protective plate in the Y direction satisfies 0.8 mm ≤ W3 ≤ 2 mm. Since the tungsten plate can absorb and block most of the rays, and simultaneously transfer the heat generated by the biased analog-to-digital converter 2, normal operation of the biased analog-to-digital converter 2 is guaranteed, ensuring stability and reliability of the entire biased detector sub-module 103.

W3 may be 0.8 mm, 1 mm, 1.5 mm, 1.8 mm, 2 mm, etc. A dimension of the tungsten plate in the Y direction is adjusted as desired. In practical applications, a thickness of the protective plate 5 ensures that it can not only meet needs of protection and heat dissipation, but also be smoothly placed in the avoidance position 7.

The biased detector sub-module 103 according to some embodiments of the present disclosure further includes a connector 4 electrically connected to the circuit connection substrate 32. The connector 4 is a rigid member or a flexible member. In other embodiments, the connector 4 and the circuit connection substrate 32 form a rigid-flex board, where the rigid board part is the circuit connection substrate 32, and the flexible board part is the connector 4.

The connector 4 serves as an interface to the external device, and is electrically connected to the circuit connection substrate 32. Through the connector 4, data received in the circuit connection substrate 32 can be collected, and the collected data can be transmitted to the external device, in such a manner that the data can be formed into a CT image after correction and CT algorithm, to ensure that the biased detector sub-module 103 can realize signal and data interaction with a CT system, guaranteeing smooth flow of information.

In an embodiment, the connector 4 is the rigid member, and connection between the connector 4 and the circuit connection substrate 32 is also rigid to ensure stable connection between the connector 4 and the circuit connection substrate 32. Alternatively, the connector 4 is the flexible member which is equivalent to the rigid member when realizing the connection function. In addition, the flexible member can absorb and relieve external vibrations, improving connection stability. Also, the flexible member has more diversified application scenarios.

In an embodiment, the connector 4 is connected to the circuit connection substrate 32 through the rigid-flex board. This connection through the rigid-flex board is functionally equivalent to the traditional electrical connection and can also realize effective signal transmission.

In the biased detector sub-module 103 according to some embodiments of the present disclosure, the biased analog-to-digital converter 2 and the photoelectric conversion array 1 are disposed at a same chip, to realize a shortened signal transmission path between the biased analog-to-digital converter 2 and the photoelectric conversion array 1, and reduce the loss and interference in the signal transmission process, which is conductive to improving the response speed and signal quality of the biased detector sub-module 103 and improving the imaging quality and performance of the entire CT system.

In addition, disposing the biased analog-to-digital converter 2 and the photoelectric conversion array 1 at the same chip is also conductive to simplifying the structure of the biased detector sub-module 103, decreasing the number of connections and interfaces between components, and reducing manufacturing cost and complexity of the detector module 101.

As illustrated in FIG. 7 to FIG. 9, a detector module according to embodiments in a second aspect of the present disclosure includes a module support 102 and a plurality of biased detector sub-modules 103. The plurality of biased detector sub-modules 103 are mounted at the module support 102 and sequentially arranged in the Z direction.

The plurality of biased detector sub-modules include at least two biased detector sub-modules 103 sequentially stacked in the Z direction. For two adjacent biased detector sub-modules 103 that are stacked, a part of the mounting substrate 31 of one of the two adjacent biased detector sub-modules that is not overlapped with the circuit connection substrate 32 is stacked on the circuit connection substrate of the other of the two adjacent biased detector sub-modules.

In an example, every two adjacent biased detector sub-modules 103 includes a first biased detector sub-module 1031 and a second biased detector sub-module 1032. The second tube end 12 of the first biased detector sub-module 1031 and the first tube end 11 or the second tube end 12 of the second biased detector sub-module 1032 are continuously arranged in the Z direction.

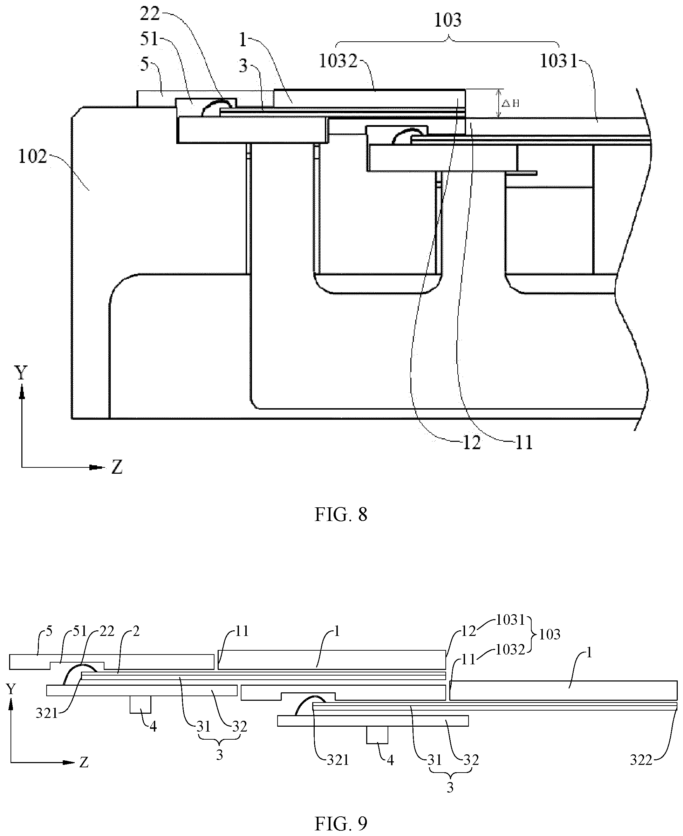

As illustrated in FIG. 9, the second tube end 12 of the first biased detector sub-module 1031 and the first tube end 11 of the second biased detector sub-module 1032 are continuously arranged in the Z direction. In this way, adjacent biased detector sub-modules 103 are ensured to be seamlessly connected in the Z direction, further ensuring that rays can be detected continuously and without interference when passing through the biased detector submodule 103.

When the second tube end 12 of the first biased detector sub-module 1031 and the first tube end 11 of the second biased detector sub-module 1032 are continuously arranged in the Z direction, the mounting substrate 31 of the first biased detector sub-module 1031 is stacked on the biased analog-to-digital converter 2 of the second biased detector sub-module 1032 in the Y direction. This stacking further improves compactness of the detector module 101 in the Y direction, and simultaneously helps to reduce the overall size and occupied space of the detector module 101.

In addition, since the mounting substrate 31 and the biased analog-to-digital converter 2 are stacked, the detector module 101 is more stable in structure, which is beneficial to improve a detection effect and reduce noise interference, improving an overall imaging effect and performance of the detector module 101.

As illustrated in FIG. 3, a detector 10 according to embodiments in a third aspect of the present disclosure includes a housing and a plurality of biased detector modules 101. The plurality of the detector modules 101 are arranged in parallel at the housing in the X direction. The detector module 101 is the detector module 101 according to the embodiments in the second aspect of the present disclosure.

The detector 10 according to the embodiments of the present disclosure can cover a wider area, capturing more image information in one scan.

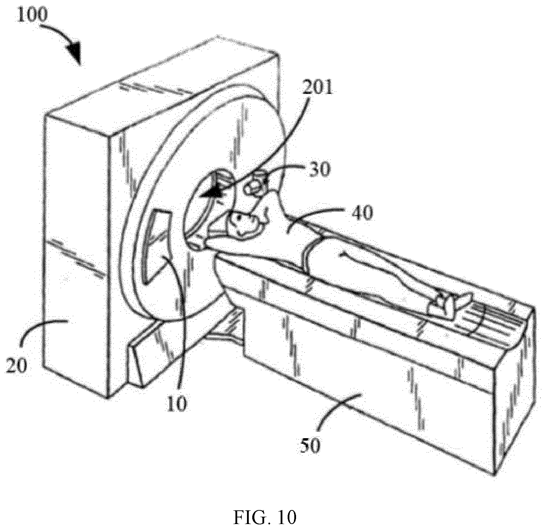

As illustrated in FIG. 10, a medical imaging device 100 according to embodiments in a fourth aspect of the present disclosure includes a scanning frame 20, a radiation source 30, and the detector 10 according to the above embodiments.

The scanning frame 20 is configured to accommodate a scanning object 40. The radiation source 30 and the detector 10 each are disposed at the scanning frame 20. The radiation source 30 is configured to emit rays to the scanning object 40. The detector 10 is configured to receive rays attenuated by the scanning object 40. When the scanning frame 20 is rotated about the Z axis, both the radiation source 30 and the detector 10 rotate with the scanning frame 20 and are maintained at positions opposite to each other in a radial direction, to enable the detector 10 to receive the rays, such as X-rays, emitted by the radiation source 30 and passing through the scanning object 40.Since the image processing capability of the detector 10 is improved, the imaging effect of the medical imaging device 100 is improved.

A structure of the scanning frame 20 is not limited. For example, the scanning frame 20 has a scanning cavity 201 configured to accommodate the scanning object 40, and the radiation source 30 and the detector 10 are disposed at two opposite sides of the scanning cavity 201 in the radial direction, respectively.

Exemplarily, the medical imaging device 100 may include, in addition to the above-described configuration, a scanning bed 50 configured to carry the scanning object 40, and of course, the present disclosure is not limited thereto. For example, when the Z direction is the vertical direction, the scanning bed 50 may not be needed. The scanning object 40 may stand upright, the scanning object 20 rotates around the Z axis while moving up and down to scan the scanning object 40, and thus details thereof will be omitted here.

A biased detector sub-module 103 according to an embodiment of the present disclosure is described below with reference to FIG. 2.

The biased detector sub-module 103 includes two photoelectric conversion arrays 1, two biased analog-to-digital converters 2, a substrate 3, a connector 4, and a protective plate 5.

The two photoelectric conversion arrays 1 are arranged in the X direction, and each of the two photoelectric conversion arrays 1 is extended in the Z direction.

The two biased analog-to-digital converters 2 are arranged in the X direction, and for each of the two biased analog-to-digital converters 2, the biased analog-to-digital converters 2 and the photoelectric conversion array 1 corresponding to the biased analog-to-digital converters 2 are arranged in the Z direction.

The substrate 3 includes the mounting substrate 31 and the circuit connection substrate 32 stacked in the Y direction.

In the Z direction, the photoelectric conversion array 1 and the biased analog-to-digital converter 2 are sequentially disposed at a side of the mounting substrate 31 facing away from the circuit connection substrate 32 to obtain support, the biased analog-to-digital converter 2 is adjacent to an end portion of the mounting substrate 31 overlapping with the circuit connection substrate 32, and a part of the mounting substrate 31 that is not overlapped with the circuit connection substrate 32 is configured to be stacked on an adjacent biased detector sub-module 103.

An electrical structure of the substrate 3 is only disposed at the circuit connection substrate 32, and the biased analog-to-digital converter 2 is electrically connected to the circuit connection substrate 32.

The connector 4 is electrically connected to the circuit connection substrate 32.

The photoelectric conversion array 1 has the first tube end 11 and the second tube end 12 in the Z direction. The biased analog-to-digital converter 2 is located adjacent to the first tube end 11.

The circuit connection substrate 32 has the first substrate end 321 and the second substrate end 322 in the Z direction. At least part of the biased analog-to-digital converter 2 is located between the first substrate end 321 and the second substrate end 322.

A distance L1 between the first substrate end 321 and the first tube end 11 in the Z direction and a distance L2 between the second substrate end 322 and the second tube end 12 in the Z direction satisfies L1 < L2.

A dimension W1 of the mounting substrate 31 in the Y direction satisfies 0.2 mm ≤ W1 ≤ 2.5 mm.

The protective plate 5 is disposed at a side of the biased analog-to-digital converter 2 facing away from the circuit connection substrate 32 and staggered from the photoelectric conversion array 1 in the Z direction.

A dimension W3 of the protective plate 5 in the Y direction satisfies 0.8 mm ≤ W3 ≤ 2 mm.

The protective plate 5 has an avoidance groove 51. A dimension W2 of the avoidance groove 51 in the Y direction satisfies 0.3 mm ≤ W2 ≤ 1 mm.

The biased analog-to-digital converter 2 includes a power connection portion 22 disposed at an end of the biased analog-to-digital converter 2 away from the photoelectric conversion array 1.

The power connection portion 22 has an end electrically connected to the biased analog-to-digital converter 2 and another end electrically connected to the circuit connection board 32.

The avoidance groove 51 at the protective plate 5 may avoid the power connection portion 22.

A detector module 101 according to an embodiment of the present disclosure is described below with reference to FIG. 7.

The detector module 101 includes a module support 102 and four biased detector sub-modules 103. The four biased detector sub-modules 103 include two first biased detector sub-modules 1031 and two second biased detector sub-modules 1032.

The first biased detector sub-module 1031 is adjacent to the second biased detector sub-module 1032.

The second tube end 12 of the first biased detector sub-module 1031 and the first tube end 11 of the second biased detector sub-module 1032 are continuously disposed in the Z direction. The mounting substrate 31 of the first biased detector sub-module 1031 is stacked on the biased analog-to-digital converter 2 of the second biased detector sub-module 1032 in the Y direction.

Other configurations of the biased detector sub-module according to the embodiments of the present disclosure, such as detectors and medical imaging devices and operations thereof are known to those of ordinary skill in the art and will not be described in detail herein.

Reference throughout this specification to terms such as "an embodiment" and "an example" means that a particular feature, structure, material, or characteristic described in connection with the embodiment or example is included in at least one embodiment or example of the present disclosure. In this specification, exemplary descriptions of aforesaid terms are not necessarily referring to the same embodiment or example. Further, the particular features, structures, materials, or characteristics may be combined in any suitable manner in one or more embodiments or examples.

Although embodiments of the present disclosure have been illustrated and described, it is conceivable for those of ordinary skill in the art that various changes, modifications, replacements, and variations can be made to these embodiments without departing from the principles of the present disclosure. The scope of the invention according to the wording of the claims shall be defined by the claims as appended.

## Claims

1. A biased detector sub-module (103), comprising:
a photoelectric conversion array (1) arranged in an X direction and a Z direction, wherein a plurality of biased detector sub-modules (103) are stacked in the Z direction to form a detector module;
a biased analog-to-digital converter (2) electrically connected to the photoelectric conversion array (1); and
a substrate (3) comprising a mounting substrate (31) and a circuit connection substrate (32) stacked in a Y direction, wherein the mounting substrate (31) has a first substrate end and a second substrate end opposite to each other in the Z direction, and the circuit connection substrate (32) has a first substrate end (321) and a second substrate end (322) opposite to each other in the Z direction, the first substrate end (321) of the circuit connection substrate (32) being adjacent to the first substrate end of the mounting substrate (31), and extending beyond the first substrate end of the mounting substrate (31), the second substrate end of the mounting substrate (31) being adjacent to the second substrate end (322) of the circuit connection substrate (32), and extending beyond the second substrate end (322) of the circuit connection substrate (32),
wherein the photoelectric conversion array (1) and the biased analog-to-digital converter (2) are sequentially disposed in the Z direction at a side of the mounting substrate (31) facing away from the circuit connection substrate (32), the biased analog-to-digital converter (2) is adjacent to the first substrate end of the mounting substrate (31), and a part of the mounting substrate (31) that is adjacent to the second substrate end of the mounting substrate (31) and that is not overlapped with the circuit connection substrate (32) is configured to be stacked on an adjacent biased detector sub-module (103),
the biased analog-to-digital converter (2) is provided with a power connection portion (22) at an end of the biased analog-to-digital converter (2) away from the photoelectric conversion array (1), the power connection portion (22) being electrically connected to a part of the circuit connection substrate (32) that is adjacent to the first substrate end (321) of circuit connection substrate (32) and that is not overlapped with the mounting substrate (31).

2. The biased detector sub-module (103) according to claim 1, wherein:
the photoelectric conversion array (1) has a first tube end (11) and a second tube end (12) in the Z direction, and the biased analog-to-digital converter (2) is located adjacent to the first tube end (11);
at least part of the biased analog-to-digital converter (2) is located between the first substrate end (321) and the second substrate end (322) of the circuit connection substrate (32); and
a distance L1 between the first substrate end (321) of the circuit connection substrate (32) and the first tube end (11) in the Z direction and a distance L2 between the second substrate end (322) of the circuit connection substrate (32) and the second tube end (12) in the Z direction satisfies L1<L2.

3. The biased detector sub-module (103) according to claim 2, wherein the mounting substrate (31) is made of a high-rigidity material.

4. The biased detector sub-module (103) according to claim 3, wherein:
the mounting substrate (31) is a ceramic substrate or a stainless steel substrate; and
a dimension W1 of the mounting substrate (31) in the Y direction satisfies 0.2 mm ≤ W1 ≤ 2.5 mm.

5. The biased detector sub-module (103) according to claim 1, further comprising a protective plate (5) disposed at a side of the biased analog-to-digital converter (2) facing away from the circuit connection substrate (32) and configured to shield radiation.

6. The biased detector sub-module (103) according to claim 5, wherein:
the protective plate (5) has an avoidance groove (51) configured to avoid the power connection portion (22).

7. The biased detector sub-module (103) according to claim 6, wherein:
a dimension W2 of the avoidance groove (51) in the Y direction satisfies 0.3 mm≤W2≤1 mm;
a dimension W3 of the protective plate (5) in the Y direction satisfies 0.8 mm ≤ W3 ≤ 2 mm.

8. The biased detector sub-module (103) according to claim 1, further comprising a connector (4) electrically connected to the circuit connection substrate (32);
the connector (4) is a rigid member or a flexible member, and the connector (4) and the circuit connection substrate (32) form a rigid-flex board.

9. The biased detector sub-module (103) according to any one of claims 1 to 8, wherein the biased analog-to-digital converter (2) and the photoelectric conversion array (1) are disposed at a same chip.

10. A detector module (101), comprising:
a module support (102); and
a plurality of biased detector sub-modules (103) according to any one of claims 1 to 8, the plurality of biased detector sub-modules (103) being mounted at the module support (102) and sequentially arranged in the Z direction;
wherein the plurality of biased detector sub-modules (103) comprise at least two biased detector sub-modules (103) sequentially stacked in the Z direction, and for two adjacent biased detector sub-modules (103) that are stacked, a part of the mounting substrate (31) of one of the two adjacent biased detector sub-modules (103) that is closer to the second substrate end of the mounting substrate (31) and that is not overlapped with the circuit connection substrate (32) is stacked on the circuit connection substrate (32) of the other of the two adjacent biased detector sub-modules (103).

11. A detector (10), comprising a housing and a plurality of detector modules (101) according to claim 10;
wherein the plurality of the detector modules (101) are arranged in parallel at the housing in the X direction.

12. A medical imaging device (100), comprising a scanning frame (20), a radiation source (30), and the detector (10) according to claim 11;
wherein the scanning frame (20) is configured to accommodate a scanning object, the radiation source (30) and the detector (10) each are disposed at the scanning frame (20), the radiation source (30) is configured to emit rays to the scanning object, and the detector (10) is configured to receive rays attenuated by the scanning object.

## Patentansprüche

1. Versetzt aufgebautes Detektorsubmodul, umfassend:
eine photoelektrische Umwandlungsanordnung (1), die in einer X-Richtung und einer Z-Richtung angeordnet ist, wobei eine Vielzahl von versetzt aufgebauten Detektorsubmodulen (103) in der Z-Richtung gestapelt sind, um ein Detektormodul zu bilden;
einen versetzt aufgebauten Analog-Digital-Wandler (2), der elektrisch mit der photoelektrischen Umwandlungsanordnung (1) verbunden ist; und
ein Substrat (3), das ein Montagesubstrat (31) und ein Schaltungsverbindungssubstrat (32) umfasst, die in einer Y-Richtung gestapelt sind, wobei das Montagesubstrat (31) ein erstes Substratende und ein zweites Substratende aufweist, die in der Z-Richtung einander gegenüberliegen, und das Schaltungsverbindungssubstrat (32) ein erstes Substratende (321) und ein zweites Substratende (322) aufweist, die in der Z-Richtung einander gegenüberliegen, wobei das erste Substratende (321) des Schaltungsverbindungssubstrats (32) benachbart zu dem ersten Substratende des Montagesubstrats (31) ist und sich über das erste Substratende des Montagesubstrats (31) hinaus erstreckt, wobei das zweite Substratende des Montagesubstrats (31) benachbart zu dem zweiten Substratende (322) des Schaltungsverbindungssubstrats (32) ist und sich über das zweite Substratende (322) des Schaltungsverbindungssubstrats (32) hinaus erstreckt,
wobei die photoelektrische Umwandlungsanordnung (1) und der versetzt aufgebaute Analog-Digital-Wandler (2) in der Z-Richtung nacheinander an einer Seite des Montagesubstrats (31) angeordnet sind, die von dem Schaltungsverbindungssubstrat (32) abgewandt ist,
wobei der versetzt aufgebaute Analog-Digital-Wandler (2) benachbart zu dem ersten Substratende des Montagesubstrats (31) ist,
und wobei ein Teil des Montagesubstrats (31), der benachbart zu dem zweiten Substratende des Montagesubstrats (31) ist und nicht mit dem Schaltungsverbindungssubstrat (32) überlappt, dazu ausgebildet ist, auf einem benachbarten versetzt aufgebauten Detektorsubmodul (103) gestapelt zu werden,
wobei der versetzt aufgebaute Analog-Digital-Wandler (2) an einem von der photoelektrischen Umwandlungsanordnung (1) abgewandten Ende des versetzt aufgebauten Analog-Digital-Wandlers (2) mit einem Stromanschlussabschnitt (22) versehen ist, wobei der Stromanschlussabschnitt (22) elektrisch mit einem Teil des Schaltungsverbindungssubstrats (32) verbunden ist, der benachbart zu dem ersten Substratende (321) des Schaltungsverbindungssubstrats (32) ist und nicht mit dem Montagesubstrat (31) überlappt.

2. Versetzt aufgebautes Detektorsubmodul (103) nach Anspruch 1, wobei:
die photoelektrische Umwandlungsanordnung (1) ein erstes Rohrende (11) und ein zweites Rohrende (12) in der Z-Richtung aufweist und der versetzt aufgebaute Analog-Digital-Wandler (2) benachbart zu dem ersten Rohrende (11) angeordnet ist;
sich mindestens ein Teil des versetzt aufgebauten Analog-Digital-Wandlers (2) zwischen dem ersten Substratende (321) und dem zweiten Substratende (322) des Schaltungsverbindungssubstrats (32) befindet; und
ein Abstand L1 zwischen dem ersten Substratende (321) des Schaltungsverbindungssubstrats (32) und dem ersten Rohrende (11) in der Z-Richtung sowie ein Abstand L2 zwischen dem zweiten Substratende (322) des Schaltungsverbindungssubstrats (32) und dem zweiten Rohrende (12) in der Z-Richtung L1<L2 erfüllen.

3. Versetzt aufgebautes Detektorsubmodul (103) nach Anspruch 2, wobei das Montagesubstrat (31) aus einem hochsteifen Material besteht.

4. Versetzt aufgebautes Detektorsubmodul (103) nach Anspruch 3, wobei:
das Montagesubstrat (31) ein Keramiksubstrat oder ein Edelstahlsubstrat ist; und
eine Abmessung W1 des Montagesubstrats (31) in Y-Richtung 0,2 mm ≤ W1 ≤ 2,5 mm erfüllt.

5. Versetzt aufgebautes Detektorsubmodul (103) nach Anspruch 1, ferner umfassend eine Schutzplatte (5), die an einer von dem Schaltungsverbindungssubstrat (32) abgewandten Seite des versetzt aufgebauten Analog-Digital-Wandlers (2) angeordnet und dazu ausgebildet ist, Strahlung abzuschirmen.

6. Versetzt aufgebautes Detektorsubmodul (103) nach Anspruch 5, wobei:
die Schutzplatte (5) eine Ausweichnut (51) aufweist, die dazu ausgebildet ist, den Stromanschlussabschnitt (22) freizustellen.

7. Versetzt aufgebautes Detektorsubmodul (103) nach Anspruch 6, wobei:
eine Dimension W2 der Ausweichnut (51) in Y-Richtung 0,3 mm ≤ W2 ≤ 1 mm erfüllt;
eine Abmessung W3 der Schutzplatte (5) in Y-Richtung 0,8 mm ≤ W3 ≤ 2 mm erfüllt.

8. Versetzt aufgebautes Detektorsubmodul (103) nach Anspruch 1, ferner umfassend einen Verbinder (4), der elektrisch mit dem Schaltungsverbindungssubstrat (32) verbunden ist,
wobei der Verbinder (4) ein starres Element oder ein flexibles Element ist und der Verbinder (4) und das Schaltungsverbindungssubstrat (32) eine Starr-flex-Leiterplatte bilden.

9. Versetzt aufgebautes Detektorsubmodul (103) nach einem der Ansprüche 1 bis 8, wobei der versetzt aufgebaute Analog-Digital-Wandler (2) und die photoelektrische Umwandlungsanordnung (1) auf demselben Chip angeordnet sind.

10. Detektormodul (101), umfassend:
einen Modulhalter (102); und
eine Vielzahl von versetzt aufgebauten Detektorsubmodulen (103) nach einem der Ansprüche 1 bis 8, wobei die Vielzahl von versetzt aufgebauten Detektorsubmodulen (103) am Modulhalter (102) montiert und in Z-Richtung nacheinander angeordnet ist,
wobei die Vielzahl von versetzt aufgebauten Detektorsubmodulen (103) mindestens zwei in der Z-Richtung nacheinander gestapelte versetzt aufgebaute Detektorsubmodule (103) umfasst, und wobei bei zwei benachbarten versetzt aufgebauten Detektorsubmodulen (103), die aufeinander gestapelt sind, ein Teil des Montagesubstrats (31) eines der beiden benachbarten versetzt aufgebauten Detektorsubmodule (103), der näher an dem zweiten Substratende des Montagesubstrats (31) liegt und nicht mit dem Schaltungsverbindungssubstrat (32) überlappt, auf dem Schaltungsverbindungssubstrat (32) des anderen der beiden benachbarten versetzt aufgebauten Detektorsubmodule (103) gestapelt ist.

11. Detektor (10), umfassend ein Gehäuse und eine Vielzahl von Detektormodulen (101) nach Anspruch 10;
wobei die Vielzahl von Detektormodulen (101) parallel in X-Richtung am Gehäuse angeordnet ist.

12. Medizinisches Bildgebungsgerät (100), umfassend einen Scanrahmen (20), eine Strahlungsquelle (30) und den Detektor (10) nach Anspruch 11;
wobei der Scanrahmen (20) dazu ausgebildet ist, ein Scanobjekt aufzunehmen, wobei die Strahlungsquelle (30) und der Detektor (10) jeweils an dem Scanrahmen (20) angeordnet sind, wobei die Strahlungsquelle (30) dazu ausgebildet ist, Strahlen auf das Scanobjekt zu emittieren, und wobei der Detektor (10) dazu ausgebildet ist, durch das Scanobjekt abgeschwächte Strahlen zu empfangen.

## Revendications

1. Un sous-module de détecteur polarisé (103), comprenant :
un réseau de conversion photoélectrique (1) agencé dans une direction X et une direction Z, dans lequel une pluralité de sous-modules de détecteur polarisés (103) sont empilés dans la direction Z pour former un module de détecteur ;
un convertisseur analogique-numérique polarisé (2) connecté électriquement au réseau de conversion photoélectrique (1) ; et
un substrat (3) comprenant un substrat de montage (31) et un substrat de connexion de circuit (32) empilés dans une direction Y, dans lequel le substrat de montage (31) présente une première extrémité de substrat et une deuxième extrémité de substrat opposées l'une à l'autre dans la direction Z, et le substrat de connexion de circuit (32) présente une première extrémité de substrat (321) et une deuxième extrémité de substrat (322) opposées l'une à l'autre dans la direction Z, la première extrémité de substrat (321) du substrat de connexion de circuit (32) étant adjacente à la première extrémité de substrat du substrat de montage (31) et s'étendant au-delà de la première extrémité de substrat du substrat de montage (31), la deuxième extrémité de substrat du substrat de montage (31) étant adjacente à la deuxième extrémité de substrat (322) du substrat de connexion de circuit (32) et s'étendant au-delà de la deuxième extrémité de substrat (322) du substrat de connexion de circuit (32),
dans lequel le réseau de conversion photoélectrique (1) et le convertisseur analogique-numérique polarisé (2) sont disposés séquentiellement dans la direction Z sur un côté du substrat de montage (31) orienté à l'opposé du substrat de connexion de circuit (32), le convertisseur analogique-numérique polarisé (2) est adjacent à la première extrémité de substrat du substrat de montage (31), et une partie du substrat de montage (31) qui est adjacente à la deuxième extrémité de substrat du substrat de montage (31) et qui n'est pas en chevauchement avec le substrat de connexion de circuit (32) est configurée pour être empilée sur un sous-module de détecteur polarisé (103) adjacent,
le convertisseur analogique-numérique polarisé (2) est pourvu d'une partie de connexion d'alimentation (22) à une extrémité du convertisseur analogique-numérique polarisé (2) éloignée du réseau de conversion photoélectrique (1), la partie de connexion d'alimentation (22) étant connectée électriquement à une partie du substrat de connexion de circuit (32) qui est adjacente à la première extrémité de substrat (321) du substrat de connexion de circuit (32) et qui n'est pas en chevauchement avec le substrat de montage (31).

2. Le sous-module de détecteur polarisé (103) selon la revendication 1, dans lequel :
le réseau de conversion photoélectrique (1) présente une première extrémité de tube (11) et une deuxième extrémité de tube (12) dans la direction Z, et le convertisseur analogique-numérique polarisé (2) est situé adjacent à la première extrémité de tube (11) ;
au moins une partie du convertisseur analogique-numérique polarisé (2) est située entre la première extrémité de substrat (321) et la deuxième extrémité de substrat (322) du substrat de connexion de circuit (32) ; et
une distance L1 entre la première extrémité de substrat (321) du substrat de connexion de circuit (32) et la première extrémité de tube (11) dans la direction Z et une distance L2 entre la deuxième extrémité de substrat (322) du substrat de connexion de circuit (32) et la deuxième extrémité de tube (12) dans la direction Z satisfont à L1<L2.

3. Le sous-module de détecteur polarisé (103) selon la revendication 2, dans lequel le substrat de montage (31) est fait d'un matériau à haute rigidité.

4. Le sous-module de détecteur polarisé (103) selon la revendication 3, dans lequel :
le substrat de montage (31) est un substrat en céramique ou un substrat en acier inoxydable ; et
une dimension W1 du substrat de montage (31) dans la direction Y satisfait à 0,2 mm ≤ W1 ≤ 2,5 mm.

5. Le sous-module de détecteur polarisé (103) selon la revendication 1, comprenant en outre une plaque de protection (5) disposée sur un côté du convertisseur analogique-numérique polarisé (2) orienté à l'opposé du substrat de connexion de circuit (32) et configurée pour blinder le rayonnement.

6. Le sous-module de détecteur polarisé (103) selon la revendication 5, dans lequel :
la plaque de protection (5) présente une rainure d'évitement (51) configurée pour éviter la partie de connexion d'alimentation (22).

7. Le sous-module de détecteur polarisé (103) selon la revendication 6, dans lequel :
une dimension W2 de la rainure d'évitement (51) dans la direction Y satisfait à 0,3 mm≤W2≤1 mm ;
une dimension W3 de la plaque de protection (5) dans la direction Y satisfait à 0,8 mm ≤ W3 ≤ 2 mm.

8. Le sous-module de détecteur polarisé (103) selon la revendication 1, comprenant en outre un connecteur (4) connecté électriquement au substrat de connexion de circuit (32) ;
le connecteur (4) est un élément rigide ou un élément flexible, et le connecteur (4) et le substrat de connexion de circuit (32) forment une carte rigide-flexible.

9. Le sous-module de détecteur polarisé (103) selon l'une quelconque des revendications 1 à 8, dans lequel le convertisseur analogique-numérique polarisé (2) et le réseau de conversion photoélectrique (1) sont disposés sur une même puce.

10. Un module de détecteur (101), comprenant :
un support de module (102) ; et
une pluralité de sous-modules de détecteur polarisés (103) selon l'une quelconque des revendications 1 à 8, la pluralité de sous-modules de détecteur polarisés (103) étant montés sur le support de module (102) et agencés séquentiellement dans la direction Z ;
dans lequel la pluralité de sous-modules de détecteur polarisés (103) comprennent au moins deux sous-modules de détecteur polarisés (103) empilés séquentiellement dans la direction Z, et pour deux sous-modules de détecteur polarisés (103) adjacents qui sont empilés, une partie du substrat de montage (31) de l'un des deux sous-modules de détecteur polarisés (103) adjacents qui est plus proche de la deuxième extrémité de substrat du substrat de montage (31) et qui n'est pas en chevauchement avec le substrat de connexion de circuit (32) est empilée sur le substrat de connexion de circuit (32) de l'autre des deux sous-modules de détecteur polarisés (103) adjacents.

11. Un détecteur (10), comprenant un boîtier et une pluralité de modules de détecteur (101) selon la revendication 10 ;
dans lequel la pluralité de modules de détecteur (101) sont agencés en parallèle au niveau du boîtier dans la direction X.

12. Un dispositif d'imagerie médicale (100), comprenant un cadre de balayage (20), une source de rayonnement (30) et le détecteur (10) selon la revendication 11 ;
dans lequel le cadre de balayage (20) est configuré pour recevoir un objet à balayer, la source de rayonnement (30) et le détecteur (10) sont chacun disposés au niveau du cadre de balayage (20), la source de rayonnement (30) est configurée pour émettre des rayons vers l'objet à balayer, et le détecteur (10) est configuré pour recevoir des rayons atténués par l'objet à balayer.
